(19) Europäisches Patentamt
European Patent Office
Office européen des brevets

(11) Publication number: 0 108 606 B1

(12) EUROPEAN PATENT SPECIFICATION

(45) Date of publication of patent specification: 26.10.88

(51) Int. Cl.$^4$: C 07 J 71/00, A 61 K 31/58

(21) Application number: 83306665.7

(22) Date of filing: 02.11.83

# (54) Steroid compounds.

(30) Priority: 02.11.82 GB 8231278

(43) Date of publication of application: 16.05.84 Bulletin 84/20

(45) Publication of the grant of the patent: 26.10.88 Bulletin 88/43

(84) Designated Contracting States: AT BE CH DE FR IT LI LU NL SE

(56) References cited:
GB-A-1 123 770
GB-A-2 010 278
US-A-3 296 255
US-A-4 196 188

CHEMICAL ABSTRACTS, vol. 88, no. 11, 13th March 1978, page 69, no. 69434d, Columbus, Ohio, US L.A. RIGG et al.: "Efficacy of intravaginal and intranasal administration of micronized 17beta-estradiol"

(73) Proprietor: STERWIN AG.
Zeughausgasse 9
CH-6300 Zug (CH)

(72) Inventor: Margetts, George
44 Broomfield Drive
Billingshurst Sussex (GB)

(74) Representative: Green, Alan James et al
SANDERSON & CO. European Patent Attorneys
34, East Stockwell Street
Colchester Essex CO1 1ST (GB)

(56) References cited:

CHEMICAL ABSTRACTS, vol. 96, no. 5, 1st February 1982, page 87, no. 28862p, Columbus, Ohio, US G.T. McINNES et al.: "Bioavailability and pharmacological activity of micronized and nonmicronized spironolactone in healthy men"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to certain steroid compounds, and in particular to pharmaceutical compositions comprising an active ingredient selected from those steroid compounds, in which the particle size of the active compound is controlled in order to obtain a higher and more acceptable level of *in vivo* activity.

The present invention is concerned specifically with 2α-cyano-4α,5α-epoxyandrostan-17β-ol-3-one having the formula:

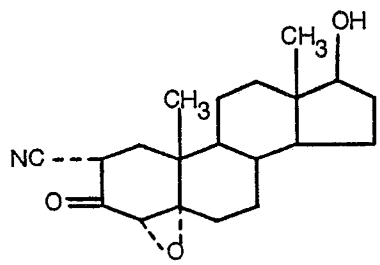

(I)

The above compound of formula (I) has the common name "trilostane" and is described, for example, in British Patent Specification No. 1,123,770 and in U.S. Patent Specification No. 3,296,255. These earlier Specifications describe the adrenocortical inhibiting properties of trilostane and related compounds, as does the article by Neumann et al in Journal of Medicinal Chemistry, *13*, 948 (1970).

It is currently proposed to use trilostane as an abortifacient, and also, more importantly, in the treatment of Cushing's Syndrome and Conn's Syndrome. Cushings's Syndrome is due to an increased secretion of hydrocortisone-type steroids, and Conn's Syndrome is due to an adenoma of the glomerulosa cells of the adrenal cortex, with which there is an increase in aldosterone secretion. Hypertension is always present in Conn's Syndrome.

It is believed that trilostane is of value in the above treatments due to its ability to act as antihypertensive, especially in those patients where hypertension is associated with high aldosterone and low renin; low aldosterone and low renin; high aldosterone and high renin; and low aldosterone and high renin.

Prior to the present invention, the use of trilostane and related compounds met with unsatisfactory results because previous formulations gave variable absoprtion characteristics and unpredictable blood levels. Thus, previous formulations of trilostane have been found to be unreliable in the treatment or control of symptoms in those conditions mentioned above due to variations in availability of trilostane *in vivo.*

Chemical Abstracts, volume 88, no. 11, 13th March, 1978, page 69, no. 79434d discloses the use of micronized 17beta-estradiol. Also, Chemical Abstracts, volume 96, no. 5, 1st February, 1982, page 87, no. 28862p discloses the use of micronized spiron.lactone. Both of those disclosures are concerned with compounds other than those defined in the present invention and, moreover, disclose micronized material as a general possibility, but apparently give no information as to what is intended by the term "micronized". In addition, the first Abstract (69434d) is concerned with routes of administration other than oral routes. Also, it is concerned with providing a dosage form for administration by those routes where absorption is quantitatively much greater than that observed after oral administration.

U.S. Specification No. 4,196,188 relates to an orally administrable form of progesterone. In accordance with the earlier invention, a therapeutically effective form of progesterone for oral administration is said to consist of particles micronised to a particle size of less than 15 microns, and such that at least 80% have a particle size of from 5 to less than about 15 microns. The earlier invention is predicated on a soft gelatin capsule pharmaceutical form containing the micronised particles in an oil vehicle, the micronised particles having been micronised in the oil phase. Furthermore, the earlier disclosure teaches the production of progesterone micronised in an oil vehicle so as to ensure that the compound remains in the micronised state to afford the stated effect after oral administration.

The teachings of the above-mentioned disclosures do not address the problems solved by the present invention in relation to preparations of trilostane or like compounds of formula II defined below. Prior to the present invention those preparations were unsatisfactory for the reasons given above and were unreliable in the treatment or control of symptoms in the kinds of conditions mentioned above due to variations in availability of, for example, trilostane *in vivo.*

However, we have now found that by processing raw trilostane and related compounds to bring their particle size within a particular narrow range, pharmaceutical compositions can be prepared which exhibit for their active ingredient improved absorption characteristics and which can avoid or substantially avoid the unsatisfactory results given by previous formulations comprising such compounds. Despite the fact that in *in vitro* testing products containing batches of trilostane of differing particle sizes could not be differentiated in respect of their dissolution or release properties, control of particle size has been shown to produce, as described in more detail below, a significant improvement in plasma concentration *in vivo.*

Accordingly, the present invention provides, in particular for use in preparing pharmaceutical compositions having improved absorption characteristics, a 2α-cyano-4α,5α-epoxy-1-oxo-steroid compound having a basic ring structure of the general formula:

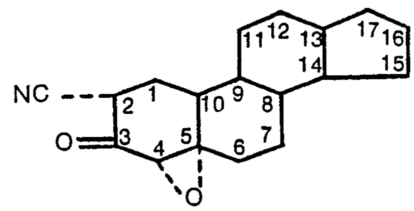

(II)

the compound being in particulate form and consisting of particles having a mean equivalent sphere volume diameter of less than about 20 μm, at least 95% of the particles having a particle size of less than about 50 μ.

In one aspect of the invention, the particulate compound may be a compound of the above formula II of the kind described in British Patent Specification No. 1,123,770, there is to say a 2α-cyano-4α,5α-epoxy-3-oxo-steroid in which the steroid moiety has from 19 to 23 carbon atoms exclusive of ester radicals.

Alternatively, in another aspect of the invention the particulate compound may be a compound of the above formula II of the kind described in British Patent Specification No. 2,010,278A, that is to say a 2α-cyano-4α,5α-epoxy-3-oxo-steroid having the general formula:

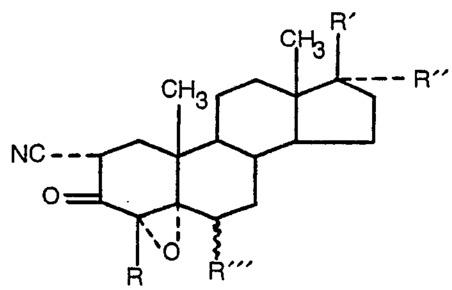

(III)

wherein R is hydrogen or methyl;

R′ is hydroxy or lower-alkanoyloxy;

R″ is hydrogen, lower-alkyl, lower-alkenyl or lower-alkynyl;

or R′ or R″ together represent oxo or ethylenedioxy;

R‴ is hydrogen or methyl;

or 3-enol lower-alkanoate esters thereof;

with the proviso that when R is hydrogen, R‴ is α-methyl, and when R is methyl, R‴ is hydrogen or β-methyl, which compounds are disclosed as being useful as interceptive agents, that is in disrupting pregnancy when administered to pregnant female mammals.

Preferably, the compound of the invention is a compound of the general formula:

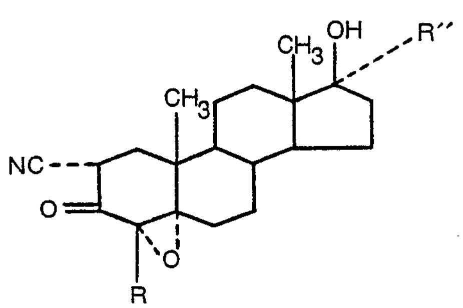

(IV)

wherein R is hydrogen or methyl and R″ is hydrogen or lower alkyl, especially methyl.

Most preferably the compound of the invention is trilostane, i.e. a compound of formula (IV) wherein R and R″ are both hydrogen; or a compound of formula (IV) wherein R and R″ are both methyl. It is believed the invention is particularly applicable to those two specific compounds, and in particular the former compound, trilostane.

Preferably, the particulate compound of the invention consists of particles having a mean equivalent sphere volume diameter of about 12 μm or less, with a preferred lower limit being about 4 to about 5. More preferably, the mean equivalent sphere volume diameter of the particles should be about 10 μm or less e.g.

from about 5 to about 10 μm, and still more preferably above 5 μm, at least 95% of particles always having a particle size of less than 50 μm.

(It will of course be understood by those familiar with comminution process techniques that the limit set on the size of 95% or more of the particles is a subsidiary limitation necessary additionally to distinguish the particulate compounds of the invention from those exhibiting a broader size distribution, because of the wide variation in size encountered in all powders reduced in size by a process of communition or particle size reduction, for example, by milling utilising a variety of kinds of milling equipment now available, for example, hammer, pin or fluid energy mills.)

The invention also provides pharmaceutical compositions comprising the said particulate compound of the invention and one or more pharmaceutically-acceptable excipients or carriers.

Such excipients or carriers may be solid, semi-solid or liquid as appropriate to the pharmaceutical form chosen, and may include a wide range of organic and inorganic solids, semi-solids and aqueous and nonaqueous liquids. Of these there may be used, for example, talc, gum arabic, starch, lactose, magnesium stearate or fatty substances of animal or vegetable origin such as cocoa butter, lanolin derivatives, paraffin derivatives or glycols. These excipients or carriers may be compounded with one or more wetting, dispersing or emulsifying agents and/or one or more preservatives as desired. Thus, the compositions of the invention may be presented as a tablet, capsule, granulate for suspension, cream, ointment, suppository or suspension.

Preferably the composition of the invention includes a solid excipient or carrier with which the said particulate compound of formula (II) is mixed, for example, starch, lactose and/or magnesium stearate.

More preferably, the composition is presented as a tablet or in an encapsulated form, the particulate compound of formula (II), together with any excipient or carrier being filled into the shell of a capsule. Preferably, the capsule contains active compound and diluent in a ratio of about 1:3.

Typically, the composition of the invention when in unit dosage form may comprise a unit dose of from about 50 to about 250 mg of the compound of formula (II), for example, about 60 mg, about 120 mg and about 240 mg, and more preferably about 50 to about 120 mg.

Preferably the particulate compound of formula (II) is also one in which the specific surface area is at least about $2 m^2 g^{-1}$ e.g. 2 to 4 $m^2 g^{-1}$.

The invention also includes a method of treating a steroid compound having a basic ring structure of the general formula (II) shown above, which method comprises processing the raw steroid compound in a treatment which reduces its particle size so as to produce a compound in particulate form and consisting of particles having a mean equivalent sphere volume diameter of less than about 20 μm, at least 95% of the particles having a particle size of less than about 50 μm.

In preparing the particulate compound of the invention a compound of formula (II), in its raw state, is first characterised for size using an instrument adapted to measure equivalent sphere volume diameter, that is to say a Coulter Counter. Typically a representative sample of a compound of formula (IV) where R and R''=H would be expected to comprise in its raw state particles having a mean equivalent sphere volume diameter of about 40 μm and with a broad size distribution.

After being characterised for size in its raw state, the raw compound is then milled, preferably using a fluid energy (air) mill, under suitable conditions e.g. of air pressure and feed rate, to bring the particle size value within the above-mentioned limits according to the invention. The efficiency of the milling is checked by sampling using a Coulter Counter and the final particle size is checked in a similar manner. If a first pass through the mill does not produce the required size spectrum, then one or more further passes are effected.

The compound of formula (II) in its particulate form within the above-mentioned limits according to the invention may then be mixed with an excipient or carrier as necessary and, for example, filled into capsules. Thus, for example, the particulate compound may be mixed with maize starch and lactose, granulated with water, dried, terminally blended with magnesium stearate, and filled into capsules.

Because the particles obtained by milling or other particle size reduction techniques are irregular in shape, it is necessary to characterise them not by measurement of an actual size such as thickness or diameter, but by measurement of a property of the particles which is related to the same property when possessed by a theoretical spherical particle. The particles are thus allocated an "equivalent sphere diameter".

The values found from characterising a large number of "unknown" particles can be plotted number vs. diameter or in other methods weight vs. diameter, usually adopting percentage oversize values for number or weight. This gives a characteristic curve representing size distribution of the sample, i.e. a cumulative percentage oversize distribution curve. Values from this can be read off directly or plotted on log-probability paper to give an approximately straight line. The mean equivalent sphere volume diameter is the 50% oversize value and the slope of the line is related to the standard deviation (s.d.) of the distribution. For convenience this may be calculated from the values at 84.1% and 15.9% oversize together with the mean equivalent sphere volume diameter thus:

$$\text{s.d.} = \frac{\text{Mean equivalent sphere volume diameter (=50\% oversize)}}{\text{Diameter at 84.1\% oversize}} \quad \text{or}$$

$$\text{s.d.} = \frac{\text{Diameter at 15.9\% oversize}}{\text{Mean equivalent sphere volume diameter (=50\% oversize)}}$$

The above applies for a log normal distribution, which may acceptably be assumed for milled power samples. Typically, a particulate compound according to the invention will give a standard deviation of about 2 µm.

The mean equivalent sphere volume diameter found is thus a statistical representation of a theoretical particle having the same property as the "unknown" particles.

As indicated above the mean equivalent sphere volume diameter of the particles of the milled compound of formula (II) may be evaluated using a Coulter Counter. Using such an instrument values for a suspension of the particles of unknown size may be obtained and the Counter may be calibrated using a standard suspension of latex particles within the size range expected for the particles of unknown size. In the case of standard latex particles values of standard deviation are extremely small because of the "sieve cut" nature or very uniform particle size.

Following is a description by way of example of the preparation of compositions in accordance with the invention. In all of the Examples the trilostane was prepared from a raw form using a fluid energy (air) mill and consisted of particles having a mean equivalent sphere volume diameter of about 10 µm, at least 95% of the particles having a particle size of less than about 50 µm. The particle size of the reduced trilostane was measured as follows:

First a small sample was checked under a microscope and a visual assessment made both of the size of the particles against a standard calibration scale, e.g. say predominantly about 10 µm, and of the uniformity of particle size. These visual assessments indicating acceptable parameters, that is most particles being within the desired size range and size distribution, a further sample of the reduced material was subjected to particle size measurement using a Coulter Counter TAII by the procedure given below. Alternatively, if the small sample visual check gave an estimated size or size distribution outside the desired specification, the milling conditions were adjusted.

The Coulter Counter particle size measurement was effected on a 5 mg sample of the reduced material, the sample being suspended in approximately 100 ml of electrolyte, for example, Isoton II. Prior to the sample suspension the electrolyte was saturated with micronised material, the pH of the resultant solution was adjusted to about 3.5, and the solution was filtered through a 0.22 µm microporous membrane filter to provide the necessary particle-free suspending electrolyte.

The electrolyte solution contained one drop of Coulter dispersant and dispersion was effected using an ultrasonic bath treatment for approximately one minute. The suspension was then diluted to approximately 200 ml with Isoton II and treated again in the ultrasonic bath for approximately one minute. The acceptability of the dispersion concentration was checked using a preset count of 70,000 particles with an aperture of 140 µm (satisfactory for 5 to 10 µm mean equivalent sphere volume diameter particles) to give a reading of less than 10% on the calibration scale. (In any size analysis technique, because the particles are of differing size, it is necessary to characterise a large number for statistical accuracy of the result. The value of 70,000 is that chosen in this case.

The tube aperture in the Coulter Counter is chosen depending on the expected particle size values. Greatest result accuracy is obtained when the particle diameters are from 4 to 40% of the aperture size to avoid false counts and disturbances. A calibration scale of 10% is a Coulter Electronics recommendation of no significance to the size).

Within ten minutes of the preparation of the dispersion, duplicate counts were performed. Duplicate counts are effected as a minimum check a) to produce more reliable measurements and b) to check that equipment sampling of the suspended material has been reproducible, i.e. the suspension has not settled.

The checked results were automatically recorded and displayed graphically to give a cumulative percentage oversize versus size characteristic curve for the sample. From this, the mean equivalent sphere volume diameter value was derived (50% oversize value) together with the standard deivation of the distribution calculated as described above.

The standard deviation was the expected value of approximately 2.0 µm, the limit being between about 1.8 µm and about 2.5 µm on ten batches.

The Coulter Counter was calibrated as follows:

A suspension of standard Coulter Electronics latex particles of stated mean diameter of 13.7 µm was treated as above using an aperture and equipment settings as described. The mean value derived was between 12.5 and 14.5 µm when the Counter was correctly calibrated.

Compositions were then prepared as follows:

Example 1

| Capsules: | Formulations | 60 mg | 120 mg |
|---|---|---|---|
| | Trilostane | 60 mg | 120 mg |
| | Lactose | 86 mg | 172 mg |
| | Maize starch | 86.65 mg | 173.3 mg |
| | Magnesium stearate | 2.35 mg | 4.7 mg |
| | | 235 mg | 470 mg |

Preparation

These formulations are prepared by either conventional dry blending techniques or more preferably conventional wet granulation techniques. Encapsulation is effected by means of conventional equipment.

Example 2

| Tablets: | Formulations | 60 mg | 120 mg | 240 mg |
|---|---|---|---|---|
| | Trilostane | 60 mg | 120 mg | 240 mg |
| | Lactose | 80 mg | 160 mg | 320 mg |
| | Maize starch | 85 mg | 170 mg | 340 mg |
| | Magnesium stearate | 2 mg | 4 mg | 8 mg |
| | | 227 mg | 454 mg | 908 mg |

Preparation

The powders are granulated by means of conventional wet granulation techniques. The tablets are compressed using conventional compression equipment to tablet or caplet form.

Example 3

| Suppositories: | Formulations | 60 mg | 120 mg | 240 mg |
|---|---|---|---|---|
| | Trilostane | 60 mg | 120 mg | 240 mg |
| | Suppository base | qs | qs | qs |

Preparation

The suppositories are prepared from a suitable suppository base (e.g. Suppocire, Witepsol, Novata, etc.) using conventional poured melt techniques or cold compression.

Example 4

| Ointment: | Formulations | 1% | 2.5% | 5% |
|---|---|---|---|---|
| | Trilostane | 1% | 2.5% | 5% |
| | Ointment base | to 100% | to 100% | to 100% |

Preparation

The ointments are prepared by incorporating the trilostane into a suitable pre-prepared ointment base (e.g. white soft paraffin).

Example 5

| Cream: | Formulations | 1% | 2.5% | 5% |
|---|---|---|---|---|
| | Trilostane | 1% | 2.5% | 5% |
| | Cream base | to 100% | to 100% | to 100% |

Preparation

The creams are prepared by incorporating the trilostance into a suitable pre-prepared cream base (e.g. aqueous cream B.P.)

Example 6

| Suspension: | Formulations | 1.2% | 2.4% | 4.8% |
|---|---|---|---|---|
| | Trilostane | 1.2% | 2.4% | 4.8% |
| | Citric acid (anhydrous) BP | 0.237% | 0.237% | 0.237% |
| | Sorbitol solution BPC | 20% | 20% | 20% |
| | Cetomacrogol 1000 BPC | 0.09% | 0.09% | 0.09% |
| | Sodium phosphate BP | 0.63% | 0.63% | 0.63% |
| | Sodium carboxy-methyl cellulose | 0.20% | 0.20% | 0.20% |
| | Veegum K | 1.00% | 1.00% | 1.00% |
| | Flavour | qs | qs | qs |
| | Preservative | qs | qs | qs |
| | Purified water | to 100% | to 100% | to 100% |

Preparation

The suspensions are prepared by conventional manufacturing techniques having regard to the processing properties of the ingredients. Finally, each suspension is passed through a homogeniser.

Bioavailability Test:

60 mg capsules were prepared as described in Example 1 by wet granulation techniques using batches of trilostane comprising (i) particles having a mean equivalent sphere volume diameter of about 10 μm (at least about 95% of the particles having a particle size of less than about 50 μm), (ii) particles of mean equivalent sphere volume diameter of about 45 μm, and (iii) particles of mean equivalent sphere volume diameter greater than 100 μm.

In a three part double blind cross-over study on eight volunteers, each volunteer received a dosage of 120 mg of trilostane (2 × 60 mg capsules) on an empty stomach with 50 ml of tap water. Blood samples (8 ml) were taken pre-medication and at 0.5, 1.0, 2.0, 3.0, 4.0, 5.0, 6.0, 8.0, 10.0 and 24.0 hours and assayed for plasma levels of trilostane and trilostane metabolite. Plasma concentrations were processed by a computer programme giving maximum concentration in plasma ($C_{max}$) and time taken to reach this maximum ($T_{max}$) by inspection. Values for the area under the curve of concentration against time over the time span 0 to t ($AUC_0^t$ — where t is time) were then calculated using the trapezoid rule and statistical analysis was carried out using the Students paired t-test.

In most cases, plasma levels could not be detected at 24 hours and consequently $AUC_0^{24}$ could be used to assess the extent of absorption. The following Table I sets out $AUC_0^{24}$ values ($mg.ml^{-1}.h$) for trilostane and 17-keto metabolite.

TABLE I

| Particle size | 10 µm | 45 µm | 100 µm |
|---|---|---|---|
| Trilostane $AUC_o^{24}$ | 1.6 | 0.6 | 0.2 |
| Metabolite $AUC_o^{24}$ | 5.0 | 2.7 | 1.3 |

As can be seen from the above results, plasma trilostane and metabolite concentration are dependent on the particle size of trilostane used, the smallest particle size material, i.e. that according to the invention, giving rise to the highest plasma concentrations. Plasma metabolite concentrations followed the time course of trilostane concentrations very closely but at a leve of 2 to 3 times higher. In addition, the ratio of Metabolite $AUC_o^{24}$ to Trilostane $AUC_o^{24}$ for the 10 µm, 45 µm and 100 µm materials is respectively 3.1, 4.5 and 6.5.

Thus, the lower $AUC_o^{24}$ values for the larger particle size capsules indicate that the extent of absorption from those capsules was reduced. Furthermore, with the smallest particle size material there is a highly favourable ratio of trilostane parent material to trilostane metabolite.

Accordingly, the results given above indicate clearly that the particle size of trilostane used in the formulations had a very pronounced influence on the availability of trilostane in plasma. The capsules containing 10 µm material resulted both in high plasma trilostane and metabolite concentrations, as well as a favourable ratio of trilostane to metabolite. However, an increase in particle size of trilostane to approximately 45 µm and then to greater than 100 µm resulted in successive reductions in the plasma concentration levels, as well as in significant increase in the ratio of trilostane metabolite to trilostane.

In our co-pending European Patent Application EP—A—0 142 309 there is described and claimed a method of preparing a compound, for example, of the above formula III in which, in particular, R is methyl, R′ is hydroxy and R″ is methyl, having a reduced particle size inter alia as defined herein, which method comprises dissolving the compound in an organic solvent, preferably dimethylformamide, precipitating the compound by mixing a non-solvent for the compound, preferably water, with the said solution, and controlling the mixing conditions to produce a precipitate having the desired reduced particle size.

It is to be understood that in preparing a compound of the formula III in accordance with the present invention in which, in particular, R is methyl, R′ is hydroxy and R″ is methyl, it is preferred to employ the method of our co-pending Application.

**Claims for the Contracting States: BE CH DE FR IT LI LU NL SE**

1. A 2α-cyano 4α,5α-epoxy-3-oxo-steroid compound, preferably having from 19 to 23 carbon atoms exclusive of ester radicals, the basic ring structure of the steroid being of the general formula:

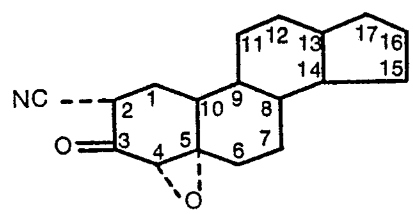

(II)

characterised in that the compound is in particulate form and consists of particles having a mean equivalent sphere volume diameter of less than about 20 µm, at least 95% of the particles having a particle size of less than about 50 µm.

2. A particulate compound according to claim 1 either of the general formula:

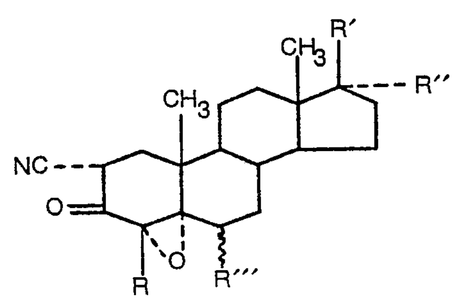

(III)

wherein R is hydrogen methyl;

R′ is hydroxy or lower-alkanoyloxy;

R″ is hydrogen, lower-alkyl, lower-alkenyl or lower-alkynyl;

or R′ and R″ together represent oxo or ethylenedioxy;
R‴ is hydrogen or methyl;
or 3-enol lower-alkanoate esters thereof;
with the proviso that when R is hydrogen, R‴ is α-methyl, and when R is methyl, R‴ is hydrogen or β-methyl,
or of the general formula:

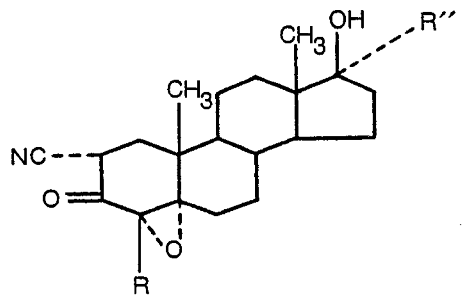

(IV)

wherein R is hydrogen or methyl and R″ is hydrogen or lower alkyl.

3. A particulate compound according to claim 2, wherein R and R″ are either both hydrogen or both methyl.

4. A particulate compound according to any one of the preceding claims, wherein the particles have a mean equivalent sphere volume diameter of about 12 μm or less, preferably about 10 μm or less.

5. A particulate compound according to claim 4, wherein the mean equivalent sphere volume diameter of the particles is from about 5 to about 10 μm, and preferably above 5 μm.

6. A particulate compound according to any one of the preceding claims, wherein its cumulative percentage oversize versus size characteristic curve exhibits a standard deviation of about 2 μm.

7. A particulate compound according to any one of the preceding claims, wherein its specific surface area is about $2m^2g^{-1}$ or higher.

8. A particulate compound according to any one of the preceding claims, wherein the mean equivalent sphere volume diameter is measured using a Coulter Counter.

9. A compound of the general formula (II) defined in claim 1 and according to any one of the preceding claims for use in preparing pharmaceutical formulations exhibiting improved absorption characteristics for parent ingredient and a higher parent ingredient to metabolite ratio.

10. A method of treating a steroid compound having a basic ring structure of the general formula II shown in claim 1, which method comprises processing the raw steroid compound in a treatment which reduces its particle size so as to produce a compound in particulate form and consisting of particles having a mean equivalent sphere volume diameter of less than about 20 μm, at least 95% of the particles having a particle size of less than about 50 μm.

11. A method according to claim 10, wherein the compound in particulate form is subsequently dry blended or wet granulated with one or more pharmaceutically-acceptable excipients or carriers, and then optionally terminally blended.

12. A pharmaceutical composition, which composition comprises a particulate compound according to any one of claims 1 to 8 and one or more pharmaceutically-acceptable excipients or carriers, preferably in the form of a tablet, a capsule, a granulate for suspension, a cream, an ointment, a suppository or a suspension, more preferably in encapsulated form, the particulate compound of formula (II), together with excipient or carrier being filled into the shell of a capsule, and the capsule containing active compound and diluent in a ratio of about 1:3.

**Claims for the Contracting State: AT**

1. A method of treating a 2α-cyano 4α,5α-epoxy-3-oxo-steroid compound, preferably having from 19 to 23 carbon atoms exclusive of ester radicals, the basic ring structure of the steroid being of the general formula:

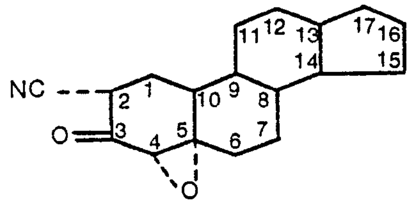

(II)

to prepare a form thereof which when formulated exhibits improved absorption characteristics, the method being characterised in that the raw compound is treated to modify its form and the treatment is controlled so that it is brought into a form which consists of particles having a mean equivalent sphere volume

diameter of less than about 20 μm, at least 95% of the particles having a particle size of less than about 50 μm.

2. A method according to claim 1, wherein the compound treated is one either of the general formula:

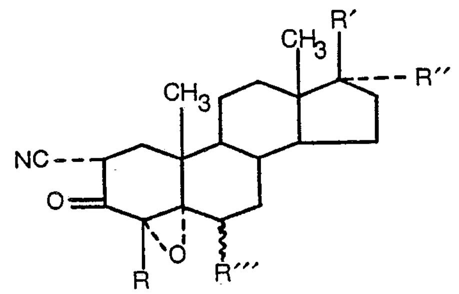

(III)

wherein R is hydrogen methyl;

R′ is hydroxy or lower-alkanoyloxy;

R″ is hydrogen, lower-alkyl, lower-alkenyl or lower-alkynyl;

or R′ and R″ together represent oxo or ethylenedioxy;

R‴ is hydrogen or methyl;

or 3-enol lower-alkanoate esters thereof;

with the proviso that when R is hydrogen, R‴ is α-methyl, and when R is methyl, R‴ is hydrogen or β-methyl,

or of the general formula:

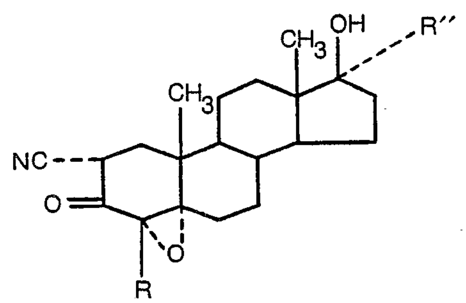

(IV)

wherein R is hydrogen or methyl and R″ is hydrogen or lower alkyl.

3. A method according to claim 2, wherein the compound treated is one in which R and R″ are either both hydrogen or both methyl.

4. A method according to any one of the preceding claims, wherein the treatment is effected so that the particles have a mean equivalent sphere volume diameter of about 12 μm or less, preferably about 10 μm or less.

5. A method according to claim 4, wherein the treatment is effected so that the mean equivalent sphere volume diameter of the particles is from about 5 to about 10 μm, and preferably above 5 μm.

6. A method according to any one of the preceding claims, wherein treatment is effected so that the cumulative percentage oversize versus size characteristic curve of the treated compound exhibits a standard deviation of about 2 μm.

7. A method according to any one of the preceding claims, wherein treatment is effected so that the specific surface area of the treated compound is about $2m^2g^{-1}$ or higher.

8. A method according to any one of the preceding claims, wherein the mean equivalent sphere volume diameter is measured using a Coulter Counter.

9. A method according to any one of the preceding claims, wherein particle size reduction is effected by employing at least one pass in a fluid energy mill.

10. A method according to any one of the preceding claims, wherein the thus-treated compound in particulate form is subsequently dry blended or wet granulated with one or more pharmaceutically-acceptable excipients or carriers, and then optionally terminally blended.

11. A 2α-cyano 4α,5α-epoxy-3-oxo-steroid compound, preferably having from 19 to 23 carbon atoms exclusive of ester radicals, the basic ring structure of the steroid being of the general formula II as shown in claim 1, characterised in that the compound is in particulate form and consists of particles having a mean equivalent sphere volume diameter of less than about 20 μm, at least 95% of the particles having a particle size of less than about 50 μm.

12. A steroid compound according to claim 11 having one or more of the compound features of any one of claims 1 to 7.

**Patentansprüche für die Vertragsstaaten: BE CH DE FR IT LI LU NL SE**

1. 2-α-Cyano-4α,5α-epoxy-3-oxo-steroidverbindung, vorzugsweise mit 19 bis 23 Kohlenstoffatomen ausgenommen Ester-Gruppen, wobei die Basis-Ringstruktur des Steroids die allgemeine Formel:

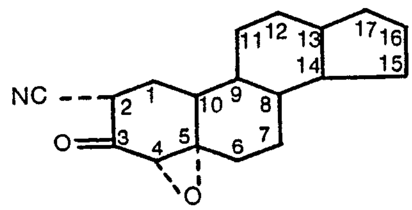

(II)

aufweist, dadurch gekennzeichnet, daß die Verbindung partikelförmig vorliegt und aus Partikeln besteht, welche einen mittleren äquivalenten Kugeldurchmesser von weniger als etwa 20 µm aufweisen, wobei mindestens 95% der Partikel eine Partikelgröße von weniger als 50 µm besitzen.

2. Partikelförmige Verbindung nach Anspruch 1 entweder gemäß der allgemeinen Formel

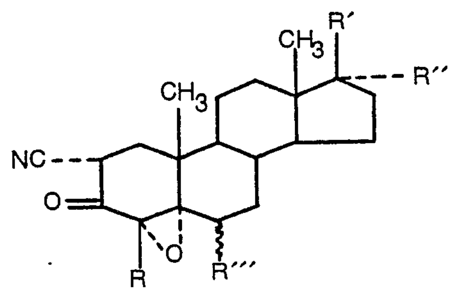

(III)

in welcher R Wasserstoff oder eine Methylgruppe ist,
R′ eine Hydroxy- oder eine niedrig-Alkanoyloxygruppe ist,
R″ Wasserstoff, eine niedrig-Alkyl-, niedrig-Alkenyl- oder niedrig-Alkynylgruppe ist
oder R′ oder R″ gemeinsam Oxo- oder Ethylendioxygruppen sind,
R‴ Wasserstoff oder eine Methylgruppe oder 3-Enol-niedrig-alkanoatester derselben ist,
mit der Maßgabe, daß R‴ oder eine α-Methylgruppe ist wenn R Wasserstoff ist und daß R‴ Wasserstoff oder eine β-Methylgruppe ist wenn R eine Methylgruppe ist,
oder gemäß der allgemeinen Formel:

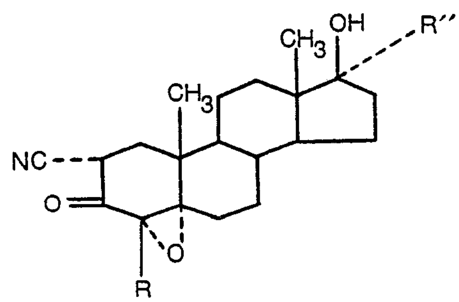

(IV)

in der R Wasserstoff oder eine Methylgruppe und R″ Wasserstoff oder eine niedrig-Alkylgruppe sind.

3. Partikelförmige Verbindung nach Anspruch 2, dadurch gekennzeichnet, daß R und R″ entweder jeweils Wasserstoff oder jeweils Methylgruppen sind.

4. Partikelförmige Verbindung nach jeglichem der vorangehenden Ansprüche, dadurch gekennzeichnet, daß die Partikel einen mitteleren äquivalenten Kugeldurchmesser von etwa 12 µm oder weniger, vorzugsweise von etwa 10 µm oder weniger aufweisen.

5. Partikelförmige Verbindung nach Anspruch 4, dadurch gekennzeichnet, daß der mittlere äquivalente Kugeldurchmesser der Partikel von etwa 5 bis etwa 10 µm und vorzugsweise mehr als 5 µm beträgt.

6. Partikelförmige Verbindung nach jeglichem der vorangehenden Ansprüche, dadurch gekennzeichnet, daß ihre cumulative prozentuale Übergröße gegenüber der charakteristischen Größenverteilung eine Standardabweichung von etwa 2 µm aufweist.

7. Partikelförmige Verbindung nach jeglichem der vorangehenden Ansprüche, dadurch gekennzeichnet, daß ihre spezifische Oberfläche etwa 2 $m^2g^{-1}$ oder mehr beträgt.

8. Partikelförmige Verbindung nach jeglichem der vorangehenden Ansprüche, dadurch gekennzeichnet, daß der mittlere äquivalente Kugeldurchmesser mit Hilfe eines Coulter Counters gemessen wird.

9. Verbindung gemäß der allgemeinen Formel II wie in Anspruch 1 definiert und nach jeglichem der vorangehenden Ansprüche zur Verwendung bei der Herstellung von pharmazeutischen Formulierungen mit verbesserten Absorptionseigenschaften für den Ursprungsbestandteil und einem höheren Verhältnis von Ursprungsbestandteil zu Metaboliten.

10. Verfahren zum Behandeln einer Steroidverbindung mit einer Basis-Ringstruktur gemäß der allgemeinen Formel II wie in Anspruch 1 dargestellt, dadurch gekennzeichnet, daß man die rohe Steroidverbindung einer Behandlung unterwirft, durch welche die Partikelgröße gesenkt wird, um auf diese Weise eine partikelförmige Verbindung herzustellen, welche aus Partikeln mit einem mittleren äquivalenten Kugeldurchmesser von weniger als etwa 20 µm besteht, wobei mindestens 95% der Partikel eine Partikelgröße von weniger als etwa 50 µm aufweisen.

11. Verfahren nach Anspruch 10, dadurch gekennzeichnet, daß die partikelförmige Verbindung nachfolgend mit einer oder mehreren pharmazeutisch verträglichen Arzneimittelzustoffen oder Trägern trocken vermischt oder feucht granuliert wird und gegenbenenfalls abschließend gemischt wird.

12. Pharmazeutische Zusammensetzung, gekennzeichnet durch den Gehalt an einer partikelförmigen Verbindung nach jeglichem der Ansprüche 1 bis 8 und einem oder mehreren pharmazeutisch verträglichen Arzneimittelzusatzstoffen oder Trägern, vorzugsweise in Form einer Tablette, einer Kapsel, eines Granulats zur Suspension, einer Creme, einer Salbe, eines Suppositoriums oder einer Suspension, in bevorzugter Weise in verkapselter Form, wobei die partikelförmige Verbindung gemäß Formel II zusammen mit dem Arzneimittelzusatzstoff oder Träger in die Schale einer Kapsel gefüllt wird und die Kapsel die aktive Verbindung und das Verschnittmittel in einem Verhältnis von etwa 1:3 enthält.

**Patentansprüche für den Vertragsstaat: AT**

1. Verfahren zum Behandeln einer 2-α-Cyano-4α,5α-epoxy-3-oxo-steroidverbindung, vorzugsweise mit 19 bis 23 Kohlenstoffatomen ausgenommen Ester-Gruppen, wobei die Basis-Ringstruktur des Steroids die allgemeine Formel:

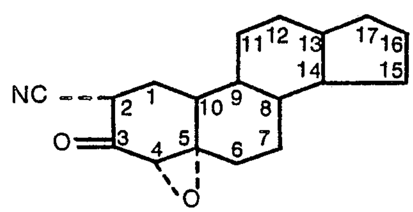

(II)

aufweist, um diese in einer Form herzustellen, die nach der Formulierung verbesserte Absorptionseingesschaften aufweist, dadurch gekennzeichnet, daß man die rohe Verbindung zur Modifikation ihrer Form behandelt und die Behandlung so steuert, daß sie in eine Form gebracht wird, die aus Partikeln besteht, welche einen mittleren äquivalenten Kugeldurchmesser von weniger als etwa 20 µm aufweisen, wobei mindestens 95% der Partikel eine Partikelgröße von weniger als 50 µm besitzen.

2. Verfahren nach Anspruch 1, dadurch gekennzeichnet, daß die behandelte Verbindung entweder die allgemeine Formel:

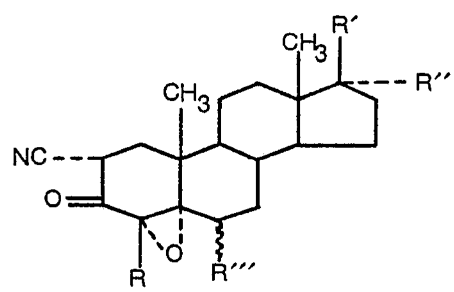

(III)

in welcher R Wasserstoff oder eine Methylgruppe ist,

R′ eine Hydroxy- oder eine niedrig-Alkanoyloxygruppe ist,

R″ Wasserstoff, eine niedrig-Alkyl-, niedrig-Alkenyl- oder niedrig-Alkynylgruppe ist

oder R′ oder R″ gemeinsam Oxo- oder Ethylendioxygruppen sind,

R‴ Wasserstoff oder eine Methylgruppe oder 3-Enol-niedrig-alkanoatester derselben ist,

mit der Maßgabe, daß R‴ oder eine α-Methylgruppe ist wenn R Wasserstoff ist und daß R‴ Wasserstoff oder eine β-Methylgruppe ist wenn R eine Methylgruppe ist,

oder die allgemeine Formel:

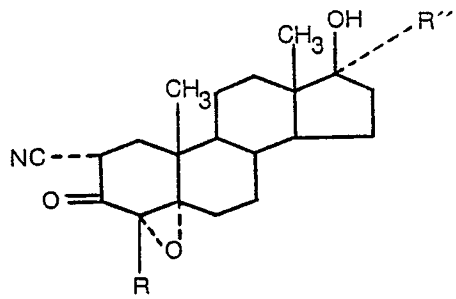

(IV)

aufweist in der R Wasserstoff oder eine Methylgruppe und R'' Wasserstoff oder eine niedrig-Alkylgruppe sind.

3. Verfahren nach Anspruch 2, dadurch gekennzeichnet, daß in der behandelten Verbindung R und R'' entweder beide Wasserstoff oder beide Methylgruppen sind.

4. Verfahren nach jeglichem der vorangehenden Ansprüche, dadurch gekennzeichnet, daß die Behandlung so ausgeführt wird, daß die Partikel einen mittleren äquivalenten Kugeldurchmesser von etwa 12 µm oder weniger, vorzugsweise von etwa 10 µm oder weniger aufweisen.

5. Verfahren nach Anspruch 4, dadurch gekennzeichnet, daß die Behandlung so ausgeführt wird, daß der mittlere äquivalente Kugeldurchmesser der Partikel von etwa 5 bis etwa 10 µm und vorzugsweise mehr als 5 µm beträgt.

6. Verfahren nach jeglichem der vorangehenden Ansprüche, dadurch gekennzeichnet, daß die Behandlung so ausgeführt wird, daß die cumulative prozentuale Übergröße gegenüber der charakteristischen Größverteilung der Partikel eine Standardabweichung von etwa 2 µm aufweist.

7. Verfahren nach jeglichem der vorangehenden Ansprüche, dadurch gekennzeichnet, daß die Behandlung so ausgeführt wird, daß die spezifische Oberfläche der behandelten Verbindung etwa 2 $m^2g^{-1}$ oder mehr beträgt.

8. Verfahren nach jeglichem der vorangehenden Ansprüche, dadurch gekennzeichnet, daß der mittlere äquivalente Kugeldurchmesser mit Hilfe eines Coulter Counters gemessen wird.

9. Verfahren nach jeglichem der vorangehenden Ansprüche, dadurch gekennzeichnet, daß die Verminderung der Partikelgröße dadurch bewirkt wird, daß man mindestens einen Durchgang mit einer Strahlmühle durchführt.

10. Verfahren nach Anspruch 10, dadurch gekennzeichnet, daß die partikelförmige Verbindung nachfolgend mit einer oder mehreren pharmazeutisch verträglichen Arzneimittelzustoffen oder Trägern trocken vermischt oder feucht granuliert wird und gegebenenfalls abschließend gemischt wird.

11. 2-α-Cyano-4α,5α-epoxy-3-oxo-steroidverbindung, vorzugsweise mit 19 bis 23 Kohlenstoffatomen ausgenommen Estergruppen, wobei die Basis-Ringstruktur des Steroids der allgemeinen Formel II wie in Anspruch 1 dargestellt entspricht, dadurch gekennzeichnet, daß die Verbindung partikelförmig ist und aus Partikeln mit einem mittleren äquivalenten Kugelvolumen von weniger als etwa 20 µm besteht, wobei mindestens 95% der Partikel eine Partikelgröße von weniger als etwa 50 µm aufweisen.

12. Steroidverbindung nach Anspruch 11, dadurch gekennzeichnet, daß sie eine oder mehrere Merkmale der Verbindungen gemäß jeglichem der Ansprüche 2 bis 7 aufweist.

**Revendications pour les Etats contractants: BE CH DE FR IT LI LU NL SE**

1. Composé 2α-cyano-4α,5α-époxy-3-oxo-stéroïdique, présenteant de préférence de 19 à 23 atomes de carbone, non compris les radicaux esters, la structure cyclique de base du stéroïde présentant la formule générale:

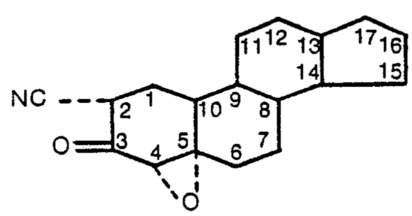

(II)

caractérisé en ce que le composé est sous forme particulaire et est constitué de particules présentant un diamètre moyen de volume sphérique équivalent inférieur à environ 20 µm, au moins 95% des particules ayant une taille de particule inférieure à environ 50 µm.

2. Composé particulaire selon la revendication 1, soit de formule générale:

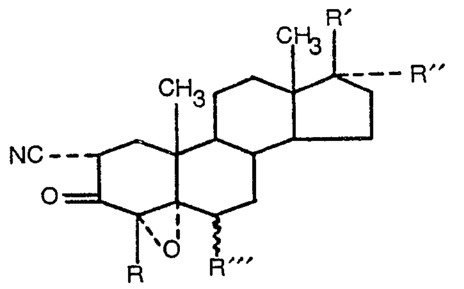

(III)

dans laquelle R est hydrogène ou méthyle;

R′ est hydroxy ou alcanoyloxy inférieur;

R″ est hydrogène, alcoyle inférieur, alcényle inférieur ou alcynyle inférieur;

ou R′ et R″ représentent ensemble oxo ou éthylènedioxy;

R‴ est hydrogène ou méthyle;

ou ses esters de type alcanoate inférieur de 3-énol;

étant entendu que lorsque R est hydrogène, R‴ est α-méthyle, et lorsque R est méthyle, R‴ est hydrogène ou β-méthyle,

soit de formule générale:

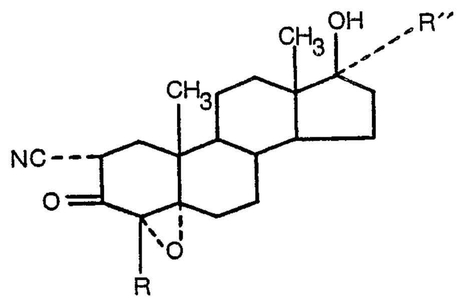

(IV)

dans laquelle R est hydrogène ou méthyle et R″ est hydrogène ou alcoyle inférieur.

3. Composé particulaire selon la revendication 2, dans lequel R et R″ sont soit l'un et l'autre hydrogène, soit l'un et l'autre méthyle.

4. Composé particulaire selon l'une quelconque des revendications précédentes, dans lequel les particules présentent un diamètre moyen de volume sphérique équivalent d'environ 12 µm ou moins, de préférence d'environ 10 µ ou moins.

5. Composé particulaire selon dans lequel le diamètre moyen de volume sphérique équivalent des particules est d'environ 5 à environ 10 µm, et de préférence supérieur à 5 µm.

6. Composé particulaire selon l'une quelconque des revendications précédentes, dans lequel son pourcentage cumulatif de surdimensions en opposition à la courbe caractéristique des dimensions présente un écart type d'environ 2 µm.

7. Composé particulaire selon l'une quelconque des revendications précédentes, dans lequel sa surface spécifique est d'environ 2 $m^2g^{-1}$ ou supérieure.

8. Composé particulaire selon l'une quelconque des revendications précédentes, dans lequel le diamètre moyen de volume sphérique équivalent est mesuré en utilisant un dispositif Coulter Counter.

9. Composé de formule générale (II) défini dans la revendication 1 et selon l'une quelconque des revendications précédentes pour l'utilisation dans la préparation de formulations pharmaceutiques présentant des caractéristiques d'absorption améliorées vis-à-vis de l'ingrédient de base et une proportion supérieure de l'ingrédient de base au métabolite.

10. Procédé pour le traitement d'un composé stéroïdique présentant une structure cyclique de base de formule générale II présentée à la revendication 1, lequel procédé comprend le façonnage du composé stéroïdique brut selon un traitement qui réduit sa taille de particules pour produire un composé sous forme particulaire et consistant en des particules présentant un diamètre moyen de volume sphérique équivalent inférieur à environ 20 µm. au moins 95%. des particules présentant une taille de particules inférieure à environ 50 µm.

11. Procédé selon la revendication 10, selon lequel le composé sous forme particulaire est ensuite mélangé à sec ou granulé à l'état humide avec un ou plusieurs excipients ou supports pharmaceutiquement acceptables, et est ensuite éventuellement mélangé pour finir.

12. Composition pharmaceutique, laquelle composition comprend un composé particulaire selon l'une quelconque des revendications 1 à 8 et un ou plusieurs excipients ou supports pharmaceutiquement acceptables, de préférence sous forme d'un comprimé, d'une capsule, d'un granulat pour suspension, d'une crème, d'un onguent, d'un suppositoire ou d'une suspension, de préférence encore sous forme encapsulée, le composé particulaire de formule (II), ensemble avec l'excipient ou le support étant introduit dans l'enveloppe d'une capsule, et la capsule contenant le composé actif et le diluant dans une proportion d'environ 1:3.

**Revendications pour l'Etat contractant: AT**

1. Procédé de traitement d'un composé 2α-cyano-4α,5α-époxy-3-oxo-stéroïdique, présentant de préférence de 19 à 23 atomes de carbone, non compris les radicaux esters, la structure cyclique de base du stéroïde présentant la formule générale:

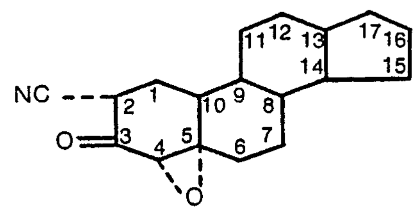

(II)

pour préparer une forme de celui-ci qui, lorsqu'elle est formulée, présente des caractéristiques d'absorption améliorées, le procédé étant caractérisé en ce que le composé brut est traité pour modifier sa forme et le traitement est réglé de sorte qu'il soit amené sous une forme qui est constituée de particules présentant un diamètre moyen de volume sphérique équivalent inférieur à environ 20 μm, au moins 95% des particules ayant une taille de particule inférieure à environ 50 μm.

2. Procédé selon la revendication 1, dans lequel le composé traité est un composé soit de formule générale:

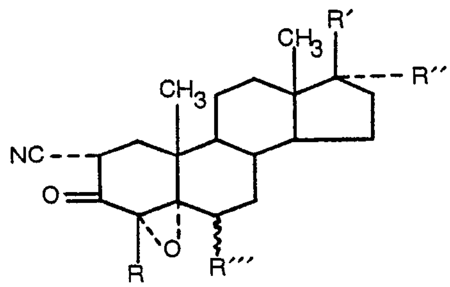

(III)

dans laquelle R est hydrogène ou méthyle;
R' est hydroxy ou alcanoyloxy inférieur;
R'' est hydrogène, alcoyle inférieur, alcényle inférieur ou alcynyle inférieur;
ou R' et R'' présentent ensemble oxo ou éthylènedioxy;
R''' est hydrogène ou méthyle;
ou ses esters de type alcanoate inférieur de 3-énol;
étant entendu que lorsque R est hydrogène, R''' est α-méthyle, et lorsque R est méthyle, R''' est hydrogène ou β-méthyle,
soit de formule générale:

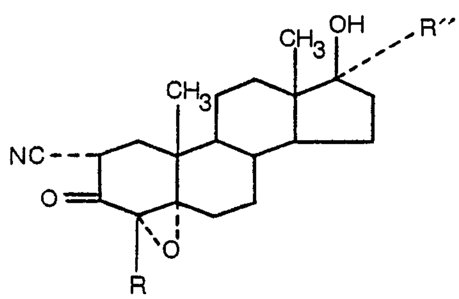

(IV)

dans laquelle R est hydrogène ou méthyle et R'' est hydrogène ou alcoyle inférieur.

3. Procédé selon la revendication 2, dans lequel le composé traité est un composé dans lequel R et R'' sont soit l'un et l'autre hydrogène, soit l'un et l'autre méthyle.

4. Procédé selon l'une quelconque des revendications précédentes, dans lequel le traitement est effectué de telle sorte que les particules présentent un diamètre moyen de volume sphérique équivalent d'environ 12 μm ou moins, de préférence d'environ 10 μm ou moins.

5. Procédé selon la revendication 4, dans lequel le traitement est effectué de telle sorte que le diamètre moyen de volume sphérique équivalent des particles est d'environ 5 à environ 10 μm, et de préférence supérieure à 5 μm.

6. Procédé selon l'une quelconque des revendications précédentes, dans lequel le traitement est effectué de telle sorte que le pourcentage cumulatif de surdimensions en opposition à la courbe caractéristique des dimensions du composé traité présente un écart type d'environ 2 μm.

7. Procédé selon l'une quelconque des revendications précédentes, dans lequel le traitement est effectué de telle sorte que la surface spécifique du composé traité soit d'environ 2 $m^2g^{-1}$ ou supérieure.

8. Procédé selon l'une quelconque des revendications précédentes, dans lequel le diamètre moyen de volume sphérique équivalent est mesuré en utilisant un dispositif Coulter Counter.

9. Procédé selon l'une quelconque des revendications précédentes, selon lequel la réduction de la taille des particules est effectuée en employant au moins un passage dans un désintégrateur à jet de fluide.

10. Procédé selon l'une quelconque des revendications précédentes, selon lequel le composé ainsi traité sous forme particulaire est ensuite mélangé à sec ou granulé à l'état humide avec un ou plusieurs excipients ou supports pharmaceutiquement acceptables, et est ensuite éventuellement mélangé pour finir.

11. Composé 2α-cyano-4α,5α-époxy-3-oxo-stéroïdique, présentant de préférence de 19 à 23 atomes de carbone, non compris les radicaux esters, la structure cyclique de base du stéroïde présentant la formule générale II représentée à la revendication 1, caractérisé en ce que le composé est sous forme particulaire et est constitué de particules présentant un diamètre moyen de volume sphérique équivalent inférieur à environ 20 µm, au moins 95% des particules ayant une taille de particule inférieure à environ 50 µm.

12. Composé stéroïdique selon la revendication 11 présentant une ou plusieurs des caractéristiques de composé de l'une quelconque des revendications 2 à 7.